# EUROPEAN PATENT SPECIFICATION

(11) **EP 3 153 081 B1**
(45) Date of publication and mention of the grant of the patent: **04.07.2018**
(21) Application number: 16193090.4
(22) Date of filing: 10.10.2016
(51) Int. Cl.: A47K 5/12, A61L 2/00

(54) **DISPENSING DEVICE FOR DISPENSING A LIQUID, SUCH AS A DISINFECTANT LIQUID, CONTAINED IN A LIQUID CONTAINER**
SPENDERVORRICHTUNG ZUM ABGEBEN EINER FLÜSSIGKEIT, INSBESONDERE EINES DESINFEKTIONSMITTELS ENTHALTEN IN EINEM FLÜSSIGKEITSBEHÄLTER
DISPOSITIF DE DISTRIBUTION POUR DISTRIBUER UN LIQUIDE, COMME UN LIQUIDE DÉSINFECTANT, CONTENU DANS UN RÉCIPIENT

(30) Priority: 09.10.2015 NL 2015593
(43) Date of publication of application: 12.04.2017
(73) Proprietor: NoSoapCompany B.V., 1221 GJ Hilversum (NL)
(72) Inventor: Voorhuis, Timo, 1221 GJ Hilversum (NL)
(74) Representative: Algemeen Octrooi- en Merkenbureau B.V.

(56) References cited:
- DE-A1- 10 103 890
- DE-B1- 2 750 936
- US-A1- 2002 074 349

## Description

The present invention relates to a dispensing device for dispensing a liquid, such as a disinfectant liquid. A disinfectant liquid is for example a liquid in the form of a gel, intended to have an alcohol content in excess of 60%.

Such dispensing devices are generally known. Typically, a liquid container containing the disinfectant liquid is placed in a receiving space of the dispensing device. A user will then connect the liquid container to connecting means that are present in the device. The connecting means will engage a passage which is present in the upper side (in use) of the liquid container. The liquid in the liquid container will flow through the connecting means to a dispensing nozzle for dispensing the liquid.

US 2002/0074349 A1 discloses a container, according the preamble of claim 1, with shave gel and a dispensing device for heating and dispensing the shave gel.

DE10103890 A1 discloses a dispensing device for dispensing a hand cleaning product.

When the liquid container is empty, i.e. (nearly) all the liquid in the liquid container has already been dispensed by the dispensing device, a user can replace the liquid container. The user will to that end disconnect the liquid container from the connecting means and subsequently place a next liquid container in a similar manner as set forth above.

A drawback of the known dispensing devices is that the risk of damage to the connecting means is relatively great. The connecting means tend to sustain damage as a result of a user's frequently connecting and disconnecting liquid containers. This is undesirable, because the connecting means must then be replaced or because a new dispensing device must be acquired.

Accordingly it is an object of the present invention, according to a first aspect thereof, to provide a dispensing device as described in the introduction wherein the frequent replacement of the liquid container will not cause damage to the connecting means.

This object is achieved with a dispensing device for dispensing a liquid, such as a disinfectant liquid, contained in a liquid container, wherein the dispensing device comprises:
- a housing;
- a receiving space present in the housing for receiving the liquid container;
- connecting means present in the housing for sealingly connecting a liquid container present therein to the dispensing device, such that the liquid contained in the liquid container can be drawn from the liquid container via a passage in an upper side (in use) of the liquid container;
- at least one pressure means present in the housing and connected to the connecting means, wherein the at least one pressure means is configured so that the connecting means are movable substantially in vertical direction relative to the housing in use, and wherein the connecting means are pressed substantially in downward direction against the liquid container by the at least one pressure means in use;
- a lifting mechanism present in the receiving space, which is configured to move a liquid container placed on the lifting mechanism substantially in upward direction in use, such that the liquid container is pressed against the connecting means that are present in the housing; and
- a collecting device connected to the lifting mechanism, which collecting device is disposed under a dispensing nozzle for dispensing the liquid in use, and which is configured to collect liquid being dispensed by the dispensing nozzle, wherein the collecting device, in a first position thereof, controls the lifting mechanism such that the mechanism will be in a receiving position for receiving the liquid container, and wherein the collecting device, in a second position thereof, being the position of use, controls the lifting mechanism such that the mechanism will press a liquid container placed thereon against the connecting means that are present in the housing.

Shocks and/or alignment errors that may occur upon connecting and disconnecting the liquid container to/from the connecting means are according to the invention absorbed/offset by the pressure means. An alignment error will for example occur if the passage of the liquid container is not disposed directly below the connecting means. That is, the passage of the liquid container is not, or not entirely correctly, in alignment with the connecting means.

Such shocks and/or alignment errors may result from inexpert handling by a user. An inexpert user will not correctly place the liquid container in the receiving space, for example. Alignment errors may also result from tolerances in the components. Within the context of the present invention, the dispensing device is placed on a supporting surface. The liquid container is movable substantially in vertical direction, which implies that the liquid container is movable in the direction transversely to the surface it is supported on.

The inventor has realised that the risk of damage to the connecting means will be reduced if connecting the liquid container to the connecting means is made easier for the user. To achieve this, the dispensing device is provided with a lifting mechanism on which the liquid container can be placed. The lifting mechanism is further configured to move a liquid container placed thereon vertically upwards, such that the liquid container is connected to the connecting means.

The lifting mechanism is operated, i.e. controlled, by the collecting device that is connected thereto. The collecting device, upon being opened to a first position, controls the lifting mechanism such that it is moved to a lower position. In this position, a liquid container can be placed on the lifting mechanism. When the collecting device is closed, i.e. moved to a second position, being the position of use, the lifting mechanism is automatically controlled such that the liquid container placed thereon will press against the connecting means present in the housing.

As a result of the above, the force with which the liquid container presses against the connecting means is uniform. This reduces the risk of damage to the liquid container and/or the connecting means upon connecting and disconnecting the liquid container to/from the connecting means.

Since the liquid container is connected to the connecting means in an upright position, with the passage at the upper side, it is preferable if the connecting means are provided with a tube. This tube must be inserted into the liquid container substantially up to the bottom of the liquid container. The liquid in the liquid container is then sucked, pressed or pumped from the liquid container in the direction of a dispensing nozzle via the tube.

The liquid container is typically suitable for containing about 1 litre of liquid, for example 0.5 l - 2 l of liquid.

In one embodiment, a connecting element can be placed on the passage in the liquid container, wherein the connecting means are configured for being connected to the connecting element.

The use of a connecting element on the passage of the liquid container has the advantage that a better connection between the connecting means and the liquid container is obtained.

In another embodiment, the housing comprises a substantially horizontally oriented (in use) stop member, wherein the connecting means are connected to the stop member via the at least one pressure means, wherein the at least one pressure means presses the connecting means substantially in downward direction against the stop member in use.

It is advantageous if the connecting means are connected to the stop member via three pressure means. This provides an efficient distribution of the forces that can occur upon connection of the liquid container to the connecting means.

The three pressure means may to that end be radially distributed around the connecting means, such that the force will be efficiently distributed.

In an advantageous embodiment, the at least one pressure means comprises at least one spring element. A spring element is excellently suitable for use as a pressure means, because it by nature comprises a contracting action which can be utilised for applying pressure to the connecting means, and because the spring can be extended/compressed such that the connecting means are movable substantially in vertical direction.

In another embodiment, the at least one pressure means is configured so that the connecting means connected thereto are movable substantially in vertical direction relative to the housing in use over a distance of 1 - 10 mm, preferably 2 - 6 mm, even more preferably 2 - 3 mm. The inventor has realised that a distance of 2 - 3 mm suffices for absorbing any shocks that may occur upon connection of the liquid container to the connecting means.

This distance of 2 - 3 mm is also excellently suitable for offsetting variations in the dimension of the liquid container.

In another embodiment, the at least one pressure means is further configured so that the connecting means connected thereto are movable substantially in horizontal direction relative to the housing in use over a distance of 0 - 6 mm, preferably 0.5 - 4 mm, even more preferably 0.5. - 1 mm.

The inventor has realised that the connecting means must be movable primarily in vertical direction, the robustness of the connecting means is additionally increased, however, if the connecting means are also movable in horizontal direction relative to the housing. In this way a searching effect is provided between the passage of the liquid container and the connecting means. Such movement of the connecting means ranges from 0 - 6 mm, preferably 0.5 - 4 mm, even more preferably 0.5 - 1 mm.

In one embodiment, the lifting mechanism comprises a parallel guide for guiding a liquid container placed thereon substantially in vertical direction.

In one example thereof, the lifting mechanism is pivotally connected to the collecting device via first pivot means.

In another embodiment, the device comprises at least one rib for supporting the movement substantially in vertical direction of a liquid container that is placed on the lifting mechanism.

The present invention will be explained in more detail hereinafter, merely by way of example, with reference to the appended figures, which show exemplary embodiments of a dispensing device according to the present invention.
Figure 1 is a perspective view of the dispensing device according to the present invention;
Figure 2 is a second perspective view of the dispensing device according to the present invention.

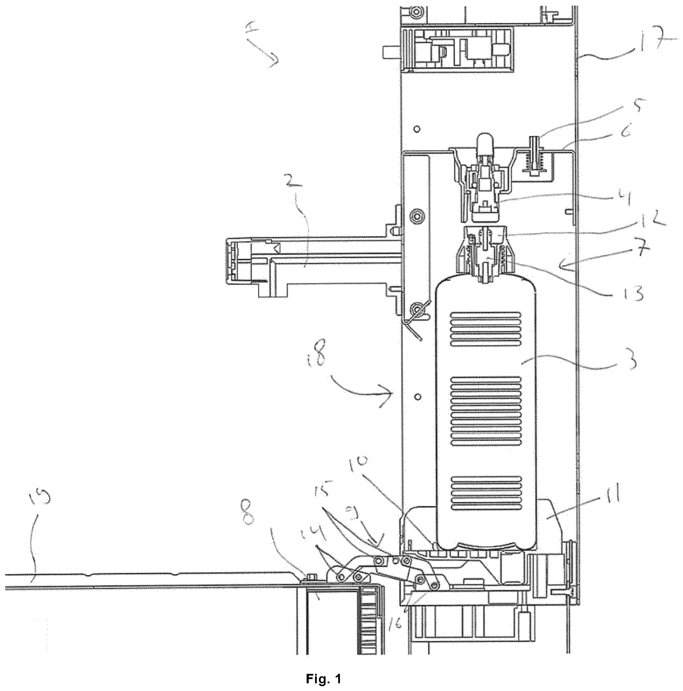

In figure 1 a perspective view is shown of the dispensing device 1 according to the present invention.

The dispensing device 1 is suitable for dispensing a liquid, such as a disinfectant liquid, contained in a liquid container 3. The disinfectant liquid is for example a gel-like substance. The liquid container 3 is typically a bottle with a volume of about 1 litre. When the bottle is empty, i.e. all the disinfectant liquid has been dispensed by the dispensing device 1, the bottle must be replaced by a user. The bottle must to that end be removed from the device 1, and a new bottle, or the same, refilled bottle, must be placed back in the device 1. This will be discussed in more detail hereinafter.

The dispensing device 1 further comprises a housing 17 and a receiving space 7 present in the housing for receiving the liquid container 3. The receiving space 7 is generally fully enclosed by the housing 17, being accessible via a front side 18 of the device 1.

The dispensing device comprises a dispensing nozzle 2 for dispensing the liquid. The liquid being dispensed is subsequently collected by a collecting device 8, which is disposed substantially below the dispensing nozzle 2 in use. In the situation shown in figure 1, the collecting device is in a first position. That is, the collecting device has been swung down. In this position, the receiving space 7 is accessible via the front side 18 of the housing 17. During the closing of the collecting device 8, i.e. the moving of the collecting device to the second position, being the position of use, the cover plate 19 is positioned against the front side 18 of the housing, so that the receiving space 7 is no longer accessible.

In the housing, connecting means 4 are furthermore present for providing a liquid-tight connection between the dispensing device 1 and a liquid container 3 placed therein. The liquid contained in the liquid container 3 can subsequently be drawn from the liquid container 3 via a passage 13 in the upper side of the liquid container 3. This can for example take place by pumping or pressurising the liquid or the like.

In order to make it easier to effect the connection or to make the connection more robust, it may be decided to place a connecting element 12 on the passage 13 before the liquid container is placed in the receiving space 7. By using this connecting element 12, a robust connection between the liquid container 3 and the connecting means 4 is ensured. The connecting element 12 may further align the liquid container 3 and the connecting means 4.

The present patent application is directed at the use of at least one pressure means 5 which is present in the housing 17 and which is connected to the connecting means 4, wherein the pressure means 5 is configured so that the connecting means 4 are movable substantially in vertical direction relative to the housing 17 in use, and wherein, in use, the at least one pressure means 5 presses the connecting means 4 substantially in downward direction against the liquid container 3 that is accommodated in the device.

The pressure means 5 comprises a spring element, for example.

In the present example, the dispensing device 1 comprises a stop member 6 which, in use, is oriented substantially horizontally, wherein the connecting means 4 are connected to the stop member 6 via the at least one pressure means 5, wherein the at least one pressure means 5 presses the connecting means 4 substantially in downward direction against the stop member 6 in use.

This solution enables the connecting means 4 to move in vertical direction relative to the stop member 6. As a result, the process of connecting the liquid container 3 to the connecting means 4 is simplified. That is, the risk of damage to one of the connecting means 4 on the liquid container 3 is strongly reduced.

In figure 1, the at least one pressure means 5 is disposed behind the connecting means 4. That is, seen from the dispensing nozzle 2, the pressure means 5 is disposed behind the connecting means 4. In order to properly distribute the force on the connecting means 4 over the stop member 6, for example, it may be advantageous if several pressure means 5 are present in the housing 17. These pressure means are in that case preferably distributed around the connecting means 4, for example on the two sides thereof, seen from the dispensing nozzle 2. The three pressure means are radially distributed around the connecting means 4, as it were.

The at least one pressure means 5 is preferably configured so that, in use, the connecting means 4 connected thereto are movable substantially in vertical direction relative to the housing 17, or the stop member 6, over a distance of 2 - 3 mm. This provides sufficient space for absorbing shocks that occur upon connecting/disconnecting the liquid container 3.

It may furthermore be preferable if the at least one pressure means 5 is also movable in horizontal direction relative to the stop member, for example over a distance of 0 - 1 mm. This has the advantageous effect that the connecting means 4 will also be movable over this distance, so that connecting the liquid container to the connecting means will be even easier.

The dispensing device 1 further comprises a lifting mechanism present in the receiving space 7, which is configured for moving a liquid container 3 placed on the lifting mechanism 9 substantially in vertically upward direction in use, such that the liquid container 3 is pressed against the connecting means 4 that are present in the housing 17.

The lifting mechanism 9 is further connected to the collecting device 8, which, in use, is disposed under the dispensing nozzle 2 for dispensing the liquid, and which is configured for collecting liquid being dispensed by the dispensing nozzle 2.

The collecting device 8 is configured so that in a first position it will control the lifting mechanism 9 such that the lifting mechanism 9 will be in a receiving position for receiving the liquid container 3, and that in a second position, being the position of use, it will control the lifting mechanism 9 such that the lifting mechanism 9 will press a liquid container 3 placed thereon against the connecting means 4 that are present in the housing.

In figure 1 the collecting device 8 is shown in the first position thereof.

The lifting mechanism 9 further comprises a parallel guide 10 for guiding a liquid container 3 placed thereon substantially in vertical direction. Furthermore, one rib 11 may be provided for supporting the movement substantially in vertical direction of a liquid container 3 placed on the lifting mechanism.

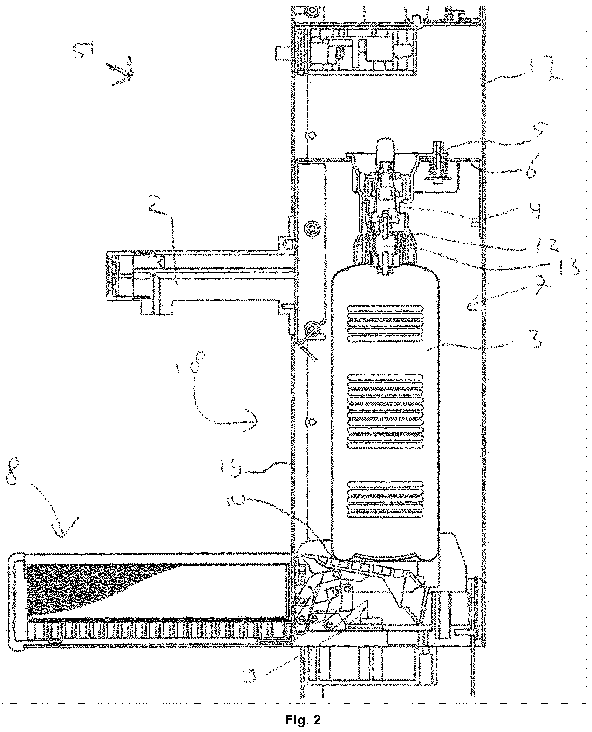

In the present example, the lifting mechanism 9 comprises a number of pivot pins 14, 15, 16. These pivot pins 14, 15, 16 are intended to apply an evenly distributed force on the liquid container 3 while the latter is being moved in vertically upward direction. The underlying idea is that the lifting mechanism 9 will press/push on the liquid container at substantially the same point. This will be apparent upon comparison of the device 1 shown in figure 1 with the device 51 shown in figure 2.

Figure 2 shows a second perspective view of the dispensing device 51 according to the present invention. In this example, the various components / aspects of the device will be indicated by the same numerals as in figure 1. This will make the figures more intelligible.

The difference between the device 51 shown in figure 2 and the device 1 shown in figure 1 is that the device 51 of figure 2 is in the position of use. That is, the device 51 is ready for use. This is because the collecting device 8 has been moved to the second position, being the position of use. In this position, the cover plate 19 is pressed against the housing 17, such that the receiving space 7 is no longer accessible from the front side 18 of the device 51.

In this position, the lifting mechanism 9 is in the upper position, such that the liquid container 3 is connected to the collecting means 4.

In the appended drawings and the above description, the present invention is shown and discussed merely with reference to a few embodiments. It will be understood that the drawings and the description are not to be construed as limiting the invention. Many variants, which may or may not obvious to the skilled person, are conceivable within the scope of the present invention as defined in the appended claims. Thus, the invention is not limited to the materials described herein. It is of course preferable that the constructional elements used for the housing and the cover plate impart sufficient strength to the dispensing device.

## Claims

1. A dispensing device (1) for dispensing a liquid, such as a disinfectant liquid, contained in a liquid container (3), wherein the dispensing device (1) comprises:
- a housing (17);
- a receiving space (7) present in the housing (17) for receiving the liquid container (3);
- connecting means (4) present in the housing (17) for sealingly connecting a liquid container (3) present therein to the dispensing device (1), such that the liquid contained in the liquid container (3) can be drawn from the liquid container (3) via a passage (13) in an upper side (in use) of the liquid container (3);
- at least one pressure means (5) present in the housing (17) and connected to the connecting means (4), wherein the at least one pressure means (5) is configured so that the connecting means (4) are movable substantially in vertical direction relative to the housing (17) in use, and wherein the connecting means (4) are pressed substantially in downward direction against the liquid container (3) by the at least one pressure means (5) in use, **characterised by**:
- a lifting mechanism (9) present in the receiving space (7), which is configured to move a liquid container (3) placed on the lifting mechanism (9) substantially in upward direction in use, such that the liquid container (3) is pressed against the connecting means (4) that are present in the housing (17);
- a collecting device (8) connected to the lifting mechanism (9), which collecting device (8) is disposed under a dispensing nozzle (2) for dispensing the liquid in use, and which is configured to collect liquid being dispensed by the dispensing nozzle (2), wherein the collecting device (8), in a first position thereof, controls the lifting mechanism (9) such that the mechanism will be in a receiving position for receiving the liquid container (3), and wherein the collecting device (8), in a second position thereof, being the position of use, controls the lifting mechanism (9) such that the mechanism will press a liquid container (3) placed thereon against the connecting means (4) that are present in the housing (17).

2. A dispensing device (1) according to claim 1, wherein a connecting element (12) can be placed on the passage (13) in the liquid container (3), and wherein the connecting means (4) are configured for being connected to the connecting element (12).

3. A dispensing device (1) according to any one of the preceding claims, wherein the housing comprises a substantially horizontally oriented (in use) stop member (6), wherein the connecting means (4) are connected to the stop member (6) via the at least one pressure means (5), wherein the at least one pressure means (5) presses the connecting means (4) substantially in downward direction against the stop member (6) in use.

4. A dispensing device (1) according to claim 3, wherein the connecting means (4) are connected to the stop member (6) via three pressure means.

5. A dispensing device (1) according to claim 4, wherein the three pressure means are radially distributed around the connecting means (4)

6. A dispensing device (1) according to any one of the preceding claims, wherein the at least one pressure means (5) comprises at least one spring element.

7. A dispensing device (1) according to any one of the preceding claims, wherein the at least one pressure means (5) is configured so that the connecting means (4) connected thereto are movable substantially in vertical direction relative to the housing (17) in use over a distance of 1 - 10 mm, preferably 2 - 6 mm, even more preferably 2 - 3 mm.

8. A dispensing device (1) according to any one of the preceding claims, wherein the at least one pressure means (5) is further configured so that the connecting means (4) connected thereto are movable substantially horizontal direction relative to the housing (17) in use over a distance of 0 - 6 mm, preferably 0.5 - 4 mm, even more preferably 0.5 - 1 mm.

9. A dispensing device (1) according to any one of the preceding claims, wherein the lifting mechanism (9) comprises a parallel guide for guiding a liquid container (3) placed thereon substantially in vertical direction.

10. A dispensing device (1) according to any one of the preceding claims, wherein the device comprises at least one rib (11) for supporting the movement substantially in vertical direction of a liquid container (3) that is placed on the lifting mechanism (9).

## Patentansprüche

1. Eine Spendervorrichtung (1) für die Abgabe einer Flüssigkeit wie einer in einem Flüssigkeitsbehälter (3) enthaltenen Desinfektionsflüssigkeit, wobei die Spendervorrichtung (1):
- ein Gehäuse (17),
- einen Aufnahmeraum (7), der in dem Gehäuse (17) vorhanden ist und den Flüssigkeitsbehälter (3) aufnimmt,
- Anschlussmittel (4), die in dem Gehäuse (17) vorhanden sind, zum dichten Verbinden des darin vorhandenen Flüssigkeitsbehälters (3) mit der Spendervorrichtung (1) derart, dass (bei Benutzung) die in dem Flüssigkeitsbehälter (3) enthaltene Flüssigkeit von dem Flüssigkeitsbehälter (3) über einen Durchlass (13) in der Oberseite des Flüssigkeitsbehälters (3) abgezogen werden kann, und
- mindestens ein Druckmittel (5), das in dem Gehäuse (17) vorhanden ist und mit den Anschlussmitteln (4) verbunden ist, wobei das mindestens eine Druckmittel (5) so gestaltet ist, dass bei Benutzung die Anschlussmittel (4) im Wesentlichen in vertikaler Richtung relativ zu dem Gehäuse (17) beweglich sind, und wobei bei Benutzung die Anschlussmittel (4) durch das mindestens eine Druckmittel (5) im Wesentlichen nach unten an den Flüssigkeitsbehälter (3) gedrückt werden, umfasst, **gekennzeichnet durch:**
- einen Hebemechanismus (9), der in dem Aufnahmeraum (7) vorhanden und so gestaltet ist, dass er bei Benutzung den auf dem Hebemechanismus (9) angeordneten Flüssigkeitsbehälter (3) im Wesentlichen nach oben derart bewegt, dass der Flüssigkeitsbehälter (3) an die in dem Gehäuse (17) vorhandenen Anschlussmittel (4) gedrückt wird, und
- eine Sammeleinrichtung (8), die mit dem Hebemechanismus (9) verbunden ist, wobei die Sammeleinrichtung (8) unter einer Auslaufdüse (2) für die Abgabe der Flüssigkeit bei Benutzung angeordnet und so gestaltet ist, dass von ihr von der Auslaufdüse (2) abgegebene Flüssigkeit gesammelt wird, wobei die Sammeleinrichtung (8) in einer ersten Position von ihr den Hebemechanismus (9) derart steuert, dass sich der Mechanismus in einer aufnehmenden Position für die Aufnahme des Flüssigkeitsbehälters (3) befindet und wobei die Sammeleinrichtung (8) in einer zweiten Position von ihr, welche die Benutzungsposition ist, den Hebemechanismus (9) derart steuert, dass durch den Mechanismus der darauf angeordnete Flüssigkeitsbehälter (3) an die in dem Gehäuse (17) vorhandenen Anschlussmittel (4) gedrückt wird.

2. Eine Spendervorrichtung (1) nach Anspruch 1, wobei ein Anschlusselement (12) auf dem Durchlass (13) in dem Flüssigkeitsbehälter (3) angeordnet werden kann und wobei die Anschlussmittel (4) so gestaltet sind, dass sie mit dem Anschlusselement (12) verbunden werden.

3. Eine Spendervorrichtung (1) nach einem der vorhergehenden Ansprüche, wobei das Gehäuse ein (bei Benutzung) im Wesentlichen horizontal ausgerichtetes Anschlagsteil (6) umfasst, wobei die Anschlussmittel (4) über das mindestens eine Druckmittel (5) mit dem Anschlagsteil (6) verbunden sind, wobei bei Benutzung das mindestens eine Druckmittel (5) die Anschlussmittel (4) im Wesentlichen nach unten an das Anschlagsteil (6) drückt.

4. Eine Spendervorrichtung (1) nach Anspruch 3, wobei die Anschlussmittel (4) über drei Druckmittel mit dem Anschlagsteil (6) verbunden sind.

5. Eine Spendervorrichtung (1) nach Anspruch 4, wobei die drei Druckmittel um die Anschlussmittel (4) herum radial verteilt sind.

6. Eine Spendervorrichtung (1) nach einem der vorhergehenden Ansprüche, wobei das mindestens eine Druckmittel (5) mindestens ein Federelement umfasst.

7. Eine Spendervorrichtung (1) nach einem der vorhergehenden Ansprüche, wobei das mindestens eine Druckmittel (5) so gestaltet ist, dass die mit ihm verbundenen Anschlussmittel (4) bei Benutzung im Wesentlichen in vertikaler Richtung relativ zu dem Gehäuse (17) über eine Entfernung von 1 bis 10 mm, vorzugsweise 2 bis 6 mm, und besonders bevorzugt 2 bis 3 mm beweglich sind.

8. Eine Spendervorrichtung (1) nach einem der vorhergehenden Ansprüche, wobei das mindestens eine Druckmittel (5) weiterhin so gestaltet ist, dass die mit ihm verbundenen Anschlussmittel (4) bei Benutzung im Wesentlichen in horizontaler Richtung relativ zu dem Gehäuse (17) über eine Entfernung von 0 bis 6 mm, vorzugsweise 0,5 bis 4 mm, und besonders bevorzugt 0,5 bis 1 mm beweglich sind.

9. Eine Spendervorrichtung (1) nach einem der vorhergehenden Ansprüche, wobei der Hebemechanismus (9) eine parallele Führung für die Führung des Flüssigkeitsbehälters (3), der darauf im Wesentlichen in vertikaler Richtung angeordnet ist, umfasst.

10. Eine Spendervorrichtung (1) nach einem der vorhergehenden Ansprüche, wobei die Vorrichtung mindestens eine Rippe (11) für die Unterstützung der Bewegung des Flüssigkeitsbehälters (3), der auf dem Hebemechanismus (9) angeordnet ist, in im Wesentlichen vertikaler Richtung umfasst.

## Revendications

1. Dispositif de distribution (1) pour distribuer un liquide, tel qu'un liquide désinfectant, contenu dans un récipient de liquide (3), dans lequel le dispositif de distribution (1) comprend :
- un boîtier (17) ;
- un espace de réception (7) présent dans le boîtier (17) pour recevoir le récipient de liquide (3) ;
- des moyens de liaison (4) présents dans le boîtier (17) pour relier de manière étanche un récipient de liquide (3) présent dans celui-ci au dispositif de distribution (1), de sorte que le liquide contenu dans le récipient de liquide (3) puisse être aspiré du récipient de liquide (3) par l'intermédiaire d'un passage (13) dans un côté supérieur (lors de l'utilisation) du récipient de liquide (3) ;
- au moins un moyen de pression (5) présent dans le boîtier (17) et relié aux moyens de liaison (4), où l'au moins un moyen de pression (5) est configuré de sorte que les moyens de liaison (4) soient mobiles essentiellement dans une direction verticale par rapport au boîtier (17) lors de l'utilisation, et où les moyens de liaison (4) sont pressés essentiellement dans une direction vers le bas contre le récipient de liquide (3) par l'au moins un moyen de pression (5) lors de l'utilisation, **caractérisé par** :
- un mécanisme de levage (9) présent dans l'espace de réception (7), qui est configuré pour déplacer un récipient de liquide (3) placé sur le mécanisme de levage (9) essentiellement dans une direction vers le haut lors de l'utilisation, de sorte que le récipient de liquide (3) soit pressé contre les moyens de liaison (4) qui sont présents dans le boîtier (17) ;
- un dispositif de collecte (8) relié au mécanisme de levage (9), lequel dispositif de collecte (8) est disposé sous une buse de distribution (2) pour distribuer le liquide lors de l'utilisation, et qui est configuré pour collecter le liquide distribué par la buse de distribution (2), où le dispositif de collecte (8), dans une première position de celui-ci, commande le mécanisme de levage (9) de sorte que le mécanisme soit dans une position de réception pour recevoir le récipient de liquide (3), et où le dispositif de collecte (8), dans une deuxième position de celui-ci, qui est la position d'utilisation, commande le mécanisme de levage (9) de sorte que le mécanisme presse un récipient de liquide (3) placé sur celui-ci contre les moyens de liaison (4) qui sont présents dans le boîtier (17).

2. Dispositif de distribution (1) selon la revendication 1, dans lequel un élément de liaison (12) peut être placé sur le passage (13) dans le récipient de liquide (3), et dans lequel les moyens de liaison (4) sont configurés pour être reliés à l'élément de liaison (12).

3. Dispositif de distribution (1) selon l'une quelconque des revendications précédentes, dans lequel le boîtier comprend un organe d'arrêt (6) orienté essentiellement horizontalement (lors de l'utilisation), où les moyens de liaison (4) sont reliés à l'organe d'arrêt (6) par l'intermédiaire de l'au moins un moyen de pression (5), où l'au moins un moyen de pression (5) presse les moyens de liaison (4) essentiellement dans une direction vers le bas contre l'organe d'arrêt (6) lors de l'utilisation.

4. Dispositif de distribution (1) selon la revendication 3, dans lequel les moyens de liaison (4) sont reliés à l'organe d'arrêt (6) par l'intermédiaire de trois moyens de pression.

5. Dispositif de distribution (1) selon la revendication 4, dans lequel les trois moyens de pression sont répartis radialement autour des moyens de liaison (4).

6. Dispositif de distribution (1) selon l'une quelconque des revendications précédentes, dans lequel l'au moins un moyen de pression (5) comprend au moins un élément de ressort.

7. Dispositif de distribution (1) selon l'une quelconque des revendications précédentes, dans lequel l'au moins un moyen de pression (5) est configuré de sorte que les moyens de liaison (4) reliés à celui-ci soient mobiles essentiellement dans une direction verticale par rapport au boîtier (17) lors de l'utilisation sur une distance de 1 à 10 mm, de préférence de 2 à 6 mm, encore plus préférablement de 2 à 3 mm.

8. Dispositif de distribution (1) selon l'une quelconque des revendications précédentes, dans lequel l'au moins un moyen de pression (5) est en outre configuré de sorte que les moyens de liaison (4) reliés à celui-ci soient mobiles essentiellement dans une direction horizontale par rapport au boîtier (17) lors de l'utilisation sur une distance de 0 à 6 mm, de préférence de 0,5 à 4 mm, encore plus préférablement de 0,5 à 1 mm.

9. Dispositif de distribution (1) selon l'une quelconque des revendications précédentes, dans lequel le mécanisme de levage (9) comprend un guide parallèle pour guider un récipient de liquide (3) placé sur celui-ci essentiellement dans la direction verticale.

10. Dispositif de distribution (1) selon l'une quelconque des revendications précédentes, dans lequel le dispositif comprend au moins une nervure (11) pour supporter le mouvement essentiellement dans la direction verticale d'un récipient de liquide (3) qui est placé sur le mécanisme de levage (9).
